# EUROPÄISCHE PATENTANMELDUNG

(11) **EP 4 585 585 A1**
(43) Veröffentlichungstag der Anmeldung: **16.07.2025**
(21) Anmeldenummer: 24151278.9
(22) Anmeldetag: 11.01.2024
(51) Int. Cl.: C07C 51/31, C07C 55/24, C07C 67/08, C07C 69/34

(54) **ZWEISTUFIGES VERFAHREN ZUR HERSTELLUNG VON 1,2,3,4-BUTANTETRACARBONSÄURETETRAALKYLESTERN**

(71) Anmelder: Evonik Oxeno GmbH & Co. KG, 45772 Marl (DE)
(72) Erfinder: GRASS, Michael, 45721 Haltern am See (DE); KRAFT, Johannes, 63067 Offenbach (DE); WOELK-FÄHRMANN, Michael, 45768 Marl (DE); VAN EICKELS, Michael, 45657 Recklinghausen (DE); BERGUP, lena, 48734 Reken (DE); BECKER, Stephan, 45770 Marl (DE)
(74) Vertreter: Evonik Patent Association

(57) **Zusammenfassung**

Gegenstand der Erfindung ist ein zweistufiges Verfahren zur Herstellung von 1,2,3,4-Butantetracarbonsäuretetraalkylestern, die Alkylgruppen mit 1 bis 6 Kohlenstoffatomen enthalten, ausgehend von Tetrahydrophthalsäureanhydrid (THPA). Das Verfahren umfasst die Oxidation von Tetrahydrophthalsäureanhydrid (THPA) und die anschließende Veresterung der dabei erhaltenen 1,2,3,4-Butantetracarbonsäure mit einem Alkohol mit 1 bis 6 Kohlenstoffatomen.

## Beschreibung

Die vorliegende Erfindung betrifft ein zweistufiges Verfahren zur Herstellung von 1,2,3,4-Butantetracarbonsäuretetraalkylestern, die Alkylgruppen mit 1 bis 6 Kohlenstoffatomen enthalten, ausgehend von Tetrahydrophthalsäureanhydrid (THPA). Das Verfahren umfasst die Oxidation von Tetrahydrophthalsäureanhydrid (THPA) und die anschließende Veresterung der dabei erhaltenen 1,2,3,4-Butantetracarbonsäure mit einem Alkohol mit 1 bis 6 Kohlenstoffatomen.

1,2,3,4-Butantetracarbonsäuretetraalkylester sind in der chemischen Industrie bekannte Ester und weisen die nachfolgende allgemeine Struktur auf 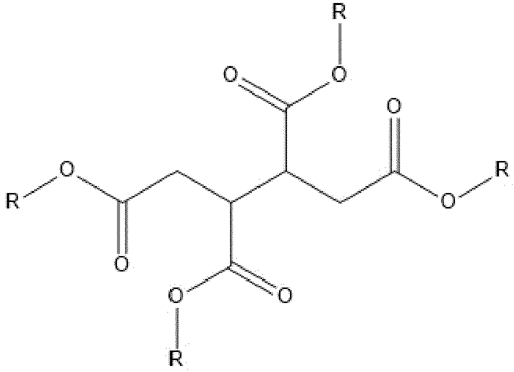 wobei alle vier Reste R jeweils für einen Alkylrest stehen. Diese Ester können beispielsweise als Weichmacher für Kunststoffe eingesetzt werden.

1,2,3,4-Butantetracarbonsäuretetraalkylester lassen sich grundsätzlich auf chemischem und elektrochemischem Wege herstellen. Die chemische Route verläuft über die Synthese von 1,2,3,4-Butantetracarbonsäure und anschließender Veresterung mit einem Alkohol zum entsprechenden 1,2,3,4-Butantetracarbonsäuretetraalkylester. Die elektrochemische Route verläuft über eine Hydrodimerisierung von Dialkylmaleat, die an der Kathode stattfindet. Derartige Verfahren sind teilweise bereits in der Patentliteratur beschrieben worden, z. B. in der EP 0 816 533 A2, in der WO 97/26389 A1 oder in der WO 02/42249 A1.

Die bekannten Verfahren weisen den Nachteil auf, dass sie großtechnisch entweder nicht wirtschaftlich oder nicht nachhaltig betrieben werden können. Weiterhin sollte eine alternative Route zur Herstellung der betreffenden 1,2,3,4-Butantetracarbonsäuretetraalkylester bereitgestellt werden.

Das erfindungsgemäße Verfahren ist ein Verfahren zur Herstellung von 1,2,3,4-Butantetracarbonsäuretetraalkylestern, die Alkylgruppen mit 1 bis 6 Kohlenstoffatome aufweisen, wobei das Verfahren die folgenden Schritte umfasst:
a) Oxidieren von Tetrahydrophthalsäureanhydrid (THPA) in wässriger Lösung mit Wasserstoffperoxid unter Verwendung eines Katalysators, wodurch eine Reaktionslösung entsteht, die zumindest 1 ,2,3,4-Butantetracarbonsäure, nicht oder nur teilweise umgesetztes THPA und restliches Wasserstoffperoxid enthält,
b) Abtrennen der 1,2,3,4-Butantetracarbonsäure aus der Reaktionslösung, die in Schritt a) erhalten wurde;
c) Verestern der 1,2,3,4-Butantetracarbonsäure mit einem C1- bis C6-Alkohol in Abwesenheit oder Gegenwart eines Katalysators;
d) Abtrennen des bei der Veresterung entstehenden Wassers und des überschüssigen Alkohols unter Erhalt der Tetraalkylester der 1 ,2,3,4-Butantetracarbonsäure mit Alkylgruppen mit 1 bis 6 Kohlenstoffatomen.

Das erfindungsgemäße Verfahren hat den Vorteil, dass bekannte und gut verfügbare Rohstoffe wie Tetrahydrophthalsäureanhydrid (THPA) und Wasserstoffperoxid eingesetzt werden. Die Herstellung von Tetrahydrophthalsäureanhydrid (THPA) erfolgt durch eine Diels-Alder-Reaktion von Maleinsäureanhydrid mit Butadien. Das vorgestellte Verfahren nach der vorliegenden Erfindung eignet sich also für die großtechnische Produktion.

Der erste Schritt a) des erfindungsgemäßen Verfahrens ist die Oxidation von Tetrahydrophthalsäureanhydrid (THPA) mit Wasserstoffperoxid. Diese Oxidation wird in wässriger Lösung unter Verwendung eines Katalysators durchgeführt und es entsteht eine Reaktionslösung, die zumindest Butantetracarbonsäure, nicht oder nur teilweise umgesetztes THPA und restliches Wasserstoffperoxid enthält. Das Reaktionsschema für die genannte Oxidation von THPA (1) zu 1,2,3,4-Butantetracarbonsäure (7) wird nachfolgend gezeigt. Bei der Oxidation werden mehrere Zwischenschritte durchlaufen, bei denen zumindest die folgenden Zwischenprodukte entstehen: 1,2,3,6-Tetrahydrophthalsäure (2), 4,5-Epoxycyclohexan-1,2-dicarbonsäure (3), 4,5-Dihydroxycyclohexan-1,2-dicarbonsäure (4), 1,5-Dipentanal-2,3-dicarbonsäure (6) und 5-Oxopentan-1,2,3-tricarbonsäure (6).

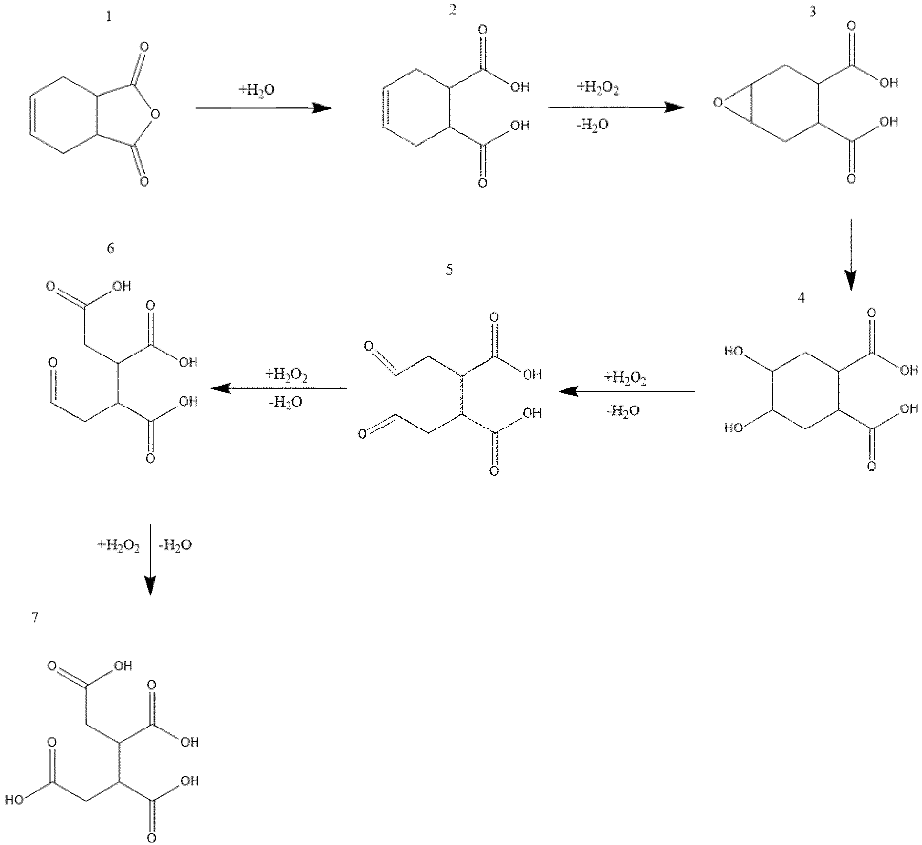

Das Tetrahydrophthalsäureanhydrid (THPA) zum Einsatz in der Oxidation nach Schritt a) ist im Handel in Reinheiten von mindestens 99.5 % verfügbar. Das Wasserstoffperoxid wird vorzugsweise als wässrige Lösung bei der Oxidation in Schritt a) eingesetzt, besonders bevorzugt wird das Wasserstoffperoxid in Form von mindestens 35 Gew.-%iger Wasserstoffperoxid-Lösung Zur Oxidation in Schritt a) gegeben.

Grundsätzlich kann das Wasserstoffperoxid in beliebiger Menge bei der Oxidation in Schritt a) eingesetzt werden, so lange die Reaktion wie gewünscht ablaufen kann. Es ist erfindungsgemäß jedoch bevorzugt, dass das Wasserstoffperoxid, insbesondere die Wasserstoffperoxid-Lösung, in einem Überschuss von 5 bis 50%, vorzugsweise 15 bis 40 %, besonders bevorzugt 20 bis 30 % zugegeben wird.

Die Oxidation in Schritt a) sollte vorzugsweise bei erhöhter Temperatur durchgeführt werden, um die Reaktion in vertretbarer Zeit ablaufen zu lassen. Es ist dabei bevorzugt, dass die Reaktionstemperatur bei der Oxidation in Schritt a) im Bereich zwischen 50 und 100 °C, vorzugsweise zwischen 70 und 98 °C, besonders bevorzugt zwischen 80 bis 95 °C liegt. Der bei der Oxidation in Schritt a) vorherrschende Druck ist weniger kritisch. Vorzugsweise wird die Oxidation in Schritt a) bei einem Druck von 0,5 bis 5 bar, vorzugsweise bei Umgebungsdruck durchgeführt.

Die Oxidation in Schritt a) wird weiterhin in Anwesenheit eines geeigneten Katalysators durchgeführt. Katalysatoren, die Oxidationsreaktionen begünstigen, sind dem Fachmann bekannt. Im Rahmen der vorliegenden Erfindung sind Katalysatoren bevorzugt, die Wolfram enthalten. Geeignete Beispiele für Wolfram-enthaltende Katalysatoren sind Phosphorwolframsäure und Natriumwolframat, von denen besonders bevorzugt Natriumwolframat eingesetzt wird. Es versteht sich, dass der Katalysator in einer katalytisch wirksamen Menge eingesetzt werden sollte. Höhere Mengen an Katalysator können auch eingesetzt werden, auch wenn das aus Kostengründen nicht sinnvoll erscheint. In einer bevorzugten Ausführungsform der vorliegenden Erfindung wird der Katalysator bei der Oxidation in Schritt a) in einer Menge von 0,01 bis 0,1 mol / 3 mol THPA eingesetzt.

Die Oxidation in Schritt a) kann sowohl kontinuierlich als auch in Batch-Fahrweise erfolgen. Der Fachmann kann die Fahrweise in Abhängigkeit der jeweiligen Gegebenheiten auswählen. Gleiches gilt für die Anlagentechnik. Die Oxidation in Schritt a) kann in einem einzigen Reaktor erfolgen, kann aber auch in mehreren parallel oder in Reihe geschalteten Reaktoren erfolgen. Geeignete Reaktoren sind dem Fachmann grundsätzlich bekannt. Ein Beispiel für einen geeigneten Reaktor für die Oxidation in Schritt a) ist ein kontinuierlicher Rührkesselreaktor.

Verfahrenstechnisch kann bei der Oxidation darauf geachtet werden, wie die einzelnen Komponenten zugegeben werden. So ist es erfindungsgemäß bevorzugt, dass das Tetrahydrophthalsäureanhydrid (THPA), der Katalysator und Wasser im Reaktor vorgelegt werden, anschließend das Gemisch aufgeheizt und gerührt und erst danach Wasserstoffperoxid zugegeben wird, wodurch die Reaktion startet.

In einer weiterhin bevorzugten Ausführungsform der vorliegenden Erfindung wird dem oder den Reaktor(en) während der Oxidation in Schritt a) des Verfahrens ein Inertgas zugeführt. Dadurch kann die Konzentration an Sauerstoff, der durch die Zersetzung von Wasserstoffperoxid entstehen kann, geringgehalten werden, um mögliche Probleme durch die Anwesenheit von Sauerstoff zu vermeiden. Als Inertgas kann jedes Gas eingesetzt werden, das sich bei der Oxidation in Schritt a) inert verhält. Bevorzugt ist das Inertgas Stickstoff, Argon oder Helium. Besonders bevorzugt ist Stickstoff. Sofern ein Inertgas eingesetzt wird, wird die bei der Oxidation entstehende Reaktionslösung zu einer Flasheinheit geleitet, um den Reaktoraustrag zu entgasen. Dabei wird vor allem das Inertgas entfernt.

Durch die Oxidation in Schritt a) wird eine Reaktionslösung erhalten, die zumindest 1,2,3,4-Butantetracarbonsäure, nicht oder nur teilweise umgesetztes THPA und restliches Wasserstoffperoxid enthält. Im nachfolgenden Schritt b) wird dann die entstandene Butantetracarbonsäure aus der Reaktionslösung abgetrennt, vorzugsweise mittels Kristallisation und anschließender Filtration. Mit dem Begriff "nur teilweise umgesetztes THPA" sind alle bei der Reaktion entstehenden Zwischenprodukte gemeint. Darunter fallen insbesondere die in dem oben gezeigten Reaktionsschema genannten Zwischenprodukte (2) bis (6).

Die Abtrennung der Butantetracarbonsäure in Schritt b) erfolgt vorzugsweise mittels Kristallation. Eine mögliche Kristallisationsmethode ist die Kühlungskristallisation, bei der die Reaktionslösung aus Schritt a) in eine geeignete Kristallisationsvorrichtung überführt und anschließend die 1,2,3,4-Butantetracarbonsäure bei einer Temperatur im Bereich von 2 bis 25 °C, vorzugsweise 3 bis 22 °C zumindest teilweise auskristallisiert wird. Bei der Kühlungskristallisation liegt vorzugsweise Umgebungsdruck vor. Durch die Kristallisation wird zumindest ein Teil der gebildeten 1,2,3,4-Butantetracarbonsäure als Feststoff ausfallen.

Eine andere Möglichkeit ist die Verdampfungskristallisation, bei der die Reaktionslösung aus Schritt a) in eine geeignete Kristallisationsvorrichtung überführt und anschließend die 1,2,3,4-Butantetracarbonsäure bei einer Temperatur im Bereich von 50 bis 70 °C, vorzugsweise 55 bis 65 °C zumindest teilweise auskristallisiert wird. Die Reaktion wird vorzugsweise im Vakuum, d. h. bei einem Druck, der geringer ist als der Umgebungsdruck, durchgeführt. Durch die Kristallisation wird zumindest ein Teil der gebildeten 1,2,3,4-Butantetracarbonsäure als Feststoff ausfallen.

Bei der Verdampfungskristallisation wird das in der Reaktionslösung befindliche Wasser zumindest teilweise verdampfen. In einer besonders bevorzugten Ausführungsform der vorliegenden Erfindung kann die Kondensationsenergie des verdampfenden Wassers genutzt werden. Dabei wird das verdampfte Wasser in einem Wärmetauscher kondensiert und die die Energie für die Verdampfung in der Kristallisationsvorrichtung eingesetzt. In einer bevorzugten Ausführungsform der vorliegenden Erfindung wird der bei der Verdampfungskristallisation entstehende Wasserdampf zur Wärmeintegration insoweit verwendet, dass zunächst eine Kompression des Wasserdampfes auf ein höheres Druckniveau erfolgt und anschließend Energie vom komprimierten Wasserdampf in einem oder mehreren Wärmetauschern innerhalb des Prozesses, z. B. bei der Kristallisation zur Erwärmung der Kristallisationslösung, zur Erwärmung des Inertgases oder zur Erwärmung beim Trocknen, übertragen wird. So muss weniger externe Energie zugeführt werden.

Unabhängig davon welches Kristallisationsverfahren angewendet wird, wird die 1,2,3,4-Butantetracarbonsäure als Feststoff in der Reaktionslösung bzw. einem Teil des Reaktionslösung vorliegen. Zur Abtrennung aus der Lösung wird vorzugsweise eine Filtration durchgeführt. Filtrationsverfahren, eventuell unter Einsatz einer Zentrifuge, sind dem Fachmann geläufig. Nach der Filtration bleibt die 1,2,3,4-Butantetracarbonsäure als Filterkuchen zurück. Die restliche Reaktionslösung wird als flüssige Phase bzw. als sogenannte Mutterlauge anfallen. Die nach der Filtration erhaltene flüssige Phase bzw. die Mutterlauge, die zumindest nicht oder nur teilweise umgesetztes THPA, den Katalysator und restliches Wasserstoffperoxid enthält, wird, ggf. nach weiteren Kristallisationsschritten, zurück zur Oxidation in Schritt a) gefahren. In der Mutterlauge sind auch die Zwischenprodukte der Oxidation nach dem oben gezeigten Reaktionsschema enthalten sein. Werden die Zwischenprodukte zurück zur Oxidation geführt, können sie zur 1,2,3,4-Butantetracarbonsäure weiterreagieren. Durch die Rückführung kann also der Gesamtumsatz erhöht werden.

Die als Filterkuchen angefallene, feste 1,2,3,4-Butantetracarbonsäure kann vor dem Einsatz in der Veresterung in Schritt c) einem Waschschritt unterworfen werden, um die Menge an restlichem Katalysator oder die Menge an sonstigen Verunreinigungen zu verringern oder vollständig zu entfernen. Zum Waschen der festen 1,2,3,4-Butantetracarbonsäure kann jede geeignete Flüssigkeit eingesetzt werden, beispielsweise Wasser oder Ethanol. Besonders bevorzugt wird Wasser zum Waschen eingesetzt, beispielsweise auch das bei der Verdampfungskristallisation verdampfte und dann wieder kondensierte Wasser.

Unabhängig davon, ob die angefallene, feste 1,2,3,4-Butantetracarbonsäure gewaschen worden ist, kann vor der Veresterung in Schritt c) eine Trocknung erfolgen. Diese Trocknung ist jedoch nicht verpflichtend. Es ist also eine Ausführungsform des erfindungsgemäßen Verfahrens denkbar, bei dem die 1,2,3,4-Butantetracarbonsäure nach der Abtrennung in Schritt b) nicht getrocknet wird bevor sie in der Veresterung in Schritt c) eingesetzt wird.

Es ist jedoch auch eine Ausführungsform des vorliegenden Verfahrens denkbar, bei dem die 1,2,3,4-Butantetracarbonsäure nach der Abtrennung in Schritt b) getrocknet wird bevor sie in der Veresterung in Schritt c) eingesetzt wird. Die Trocknung kann sowohl bei erhöhten Temperaturen durchgeführt werden als auch mittels Gefriertrocknung.

Eine gewisse Trocknung kann aber auch noch vor der Veresterung in Schritt c) dadurch erfolgen, dass der Veresterungskatalysator erst zur Veresterung in Schritt c) zugegeben wird, wenn der Wassergehalt der 1,2,3,4-Butantetracarbonsäure reduziert worden ist, vorzugsweise durch Aufheizen auf Temperaturen von größer oder gleich 70 °C, vorzugsweise größer oder gleich 100 °C.

Das Verfahren umfasst also die folgenden Ausführungsformen des Verfahrens, bei dem die Reaktionslösung einer Kühlungskristallisation und anschließend einer Filtration unterworfen wird, wobei nach der Filtration ein auskristallisiertes Produkt (feste 1,2,3,4-Butantetracarbonsäure) und eine flüssige Mutterlauge entsteht, wovon die flüssige Mutterlauge zu dem oder zu den Reaktor(en) in Schritt a) zurückgeführt wird. Vorzugsweise wird das auskristallisierte Produkt mit Wasser gewaschen und anschließend getrocknet.

In Schritt c) des erfindungsgemäßen Verfahrens wird die in Schritt a) hergestellte und in Schritt b) abgetrennte 1,2,3,4-Butantetracarbonsäure einer Veresterung mit einem C1- bis C6-Alkohol unterworfen. Dabei entsteht das gewünschte Produkt, also 1,2,3,4-Butantetracarbonsäuretetraalkylester, die Alkylgruppen mit 1 bis 6 Kohlenstoffatome aufweisen.

Der C1- bis C6-Alkohol wird bei der Veresterung in Schritt c) vorzugsweise im Überschuss eingesetzt. In einer bevorzugten Ausführungsform der vorliegenden Erfindung wird der C1- bis C6-Alkohol in einer Menge von 115 % bis 200 %, besonders bevorzugt in einer Menge von 120 % bis 150 % der zur vollständigen Veresterung notwendigen stöchiometrisch Menge eingesetzt.

Der bei der Veresterung in Schritt c) eingesetzte Alkohol ist ein C1- bis C6-Alkohol, insbesondere ein C1-bis C6-Monoalkohol, also ein Alkohol mit nur einer einzigen Alkoholgruppe. In einer bevorzugten Ausführungsform der vorliegenden Erfindung wird bei der Veresterung in Schritt c) ein C4- bis C6-Alkohol eingesetzt, wodurch 1,2,3,4-Butantetracarbonsäuretetraalkylester entstehen, die Alkylgruppen mit 4 bis 6 Kohlenstoffatome aufweisen.

Als C4- bis C6-Alkohol kann 1-Butanol, 2-Butanol, 2-Methyl-1-propanol, 2-Methyl-2-propanol, 1-Pentanol, 2-Pentanol, 3-Pentanol, 2-Methyl-1-butanol, 2-Methyl-2-butanol, 3-Methyl-1-butanol, 3-Methyl-2-butanol, 2,2-Dimethyl-1-propanol, 1-Hexanol, 2-Hexanol, 3-Hexanol, 2-Methyl-1-pentanol, 3-Methyl-1-pentanol, 4-Methyl-1-pentanol, 2-Methyl-2-pentanol, 3-Methyl-2-pentanol, 4-Methyl-2-pentanol, 2-Methyl-3-pentanol, 3-Methyl-3-pentanol, 2,2-Di-Methyl-1-butanol, 2,3-Di-Methyl-1-butanol, 3,3-Di-Methyl-1-butanol, 2,3-DiMethyl-2-butanol, 3,3-Di-Methyl-2-butanol, 3-Ethyl-1-butanol oder Mischungen von zwei oder mehr davon eingesetzt werden. Bevorzugt wird als C4- bis C6-Alkohol 1-Pentanol, 2-Pentanol, 3-Pentanol, 2-Methyl-1-butanol, 2-Methyl-2-butanol, 3-Methyl-1-butanol, 3-Methyl-2-butanol, 2,2-Dimethyl-1-propanol oder Mischungen davon eingesetzt. Besonders bevorzugt wird als C4- bis C6-Alkohol eine Mischung von mindestens zwei aus der Gruppe 1-Pentanol, 2-Methyl-1-butanol und 3-Methyl-1-butanol eingesetzt.

Die Veresterung in Schritt c) kann in Abwesenheit oder in Gegenwart eines Katalysators durchgeführt werden. Die Veresterung in Schritt c) findet vorzugsweise in Anwesenheit eines Katalysators statt. Grundsätzlich können dazu bekannte und für die Veresterung geeignete Katalysatorsysteme eingesetzt werden. Geeignete Katalysatoren für die Veresterung zur Herstellung der erfindungsgemäßen C1- bis C6-Alkylester der 1 ,2,3,4-Butantetracarbonsäure sind Titanat-Katalysatoren, beispielsweise Tetra-n-Butyltitanat, Zirkonate oder Sulfonsäuren.

Die Veresterung in Schritt c) zur Herstellung der erfindungsgemäßen Ester erfolgt bevorzugt bei einer Temperatur von 120 bis 250 °C, weiterhin bevorzugt bei einer Temperatur von 140 bis 230 °C, besonders bevorzugt bei einer Temperatur von 160 bis 215 °C. Der Druck bei der Veresterung sollte vorzugsweise nicht zu hoch sein, da dies die Siedetemperatur und dadurch auch die Veresterungstemperatur erhöhen würde. Der Druck bei der Veresterung liegt deshalb in einem Bereich von 0,5 bis 7 bar absolut, vorzugsweise bei 3 bar absolut oder weniger, besonders bevorzugt bei nicht weniger als 0,5 bar absolut. Ganz besonderes bevorzugt erfolgt die Veresterung in Schritt c) bei Umgebungsdruck.

Während einer Veresterung in Schritt c) entsteht durch die Reaktion der Säuregruppe mit dem C1 bis C6-Alkohol Wasser. Dieses Wasser wird auch als Reaktionswasser bezeichnet. In einer bevorzugten Ausführungsform der vorliegenden Erfindung wird zumindest ein Teil des entstehenden Reaktionswasser während der noch laufenden Reaktion abgetrennt. Dadurch kann u. a. das Gleichgewicht der Reaktion in die richtige Richtung verschoben werden.

Der Fortgang der Veresterungsreaktion kann durch die Beobachtung eines Parameters überwacht werden. Möglich ist beispielsweise die Überwachung der Säurezahl oder der Wassermenge. Möglich ist auch die Überwachung mittels Gaschromatographie, wo der Anteil von Edukten und/oder Produkten ermittelt werden kann. Weiterhin kann die Reaktion auch mittels Online-Analytik verfolgt werden

Ist die Reaktion ausreichend weit fortgeschritten kann die Reaktion auf unterschiedlichen Wegen beendet werden. Eine Möglichkeit ist, dass zunächst der Katalysator durch Zugabe einer Lauge zerstört wird. Gleichzeitig wird dadurch noch vorhandene Säure verseift. Anschließend kann die Reaktionslösung mit bekannten Verfahren aufgearbeitet werden. Dazu erfolgt in Schritt d) die Abtrennung des bei der Veresterung entstehenden Wassers und des überschüssigen Alkohols unter Erhalt der Tetraalkylester der Butantetracarbonsäure mit Alkylgruppen mit 1 bis 6 Kohlenstoffatomen. Diese Abtrennung erfolgt vorzugsweise mittels thermischer Trennung.

Eine andere Möglichkeit zur Beendigung der Reaktion ist, dass zunächst in Schritt d) die Abtrennung des bei der Veresterung entstehenden Wassers und des überschüssigen Alkohols unter Erhalt der Tetraalkylester der Butantetracarbonsäure mit Alkylgruppen mit 1 bis 6 Kohlenstoffatomen erfolgt. Diese Abtrennung erfolgt vorzugsweise mittels thermischer Trennung. Anschließend wird der Katalysator durch Zugabe einer Lauge zerstört.

Die gemäß der vorliegenden Erfindung erhaltenen C1- bis C5-Alkylester, vorzugsweise C4- bis C6-Alkylester, besonders bevorzugt C5-Alkylester der 1,2,3,4-Butantetracarbonsäure weisen vorteilhafte Eigenschaften beim Einsatz als Weichmacher für Polymere auf. Ein weiterer Gegenstand der vorliegenden Erfindung ist deshalb die Verwendung der C1- bis C5-Alkylester, vorzugsweise C4- bis C6-Alkylester, besonders bevorzugt C5-Alkylester der 1,2,3,4-Butantetracarbonsäure als Weichmacher für Polymere. Geeignete Polymere sind unten aufgeführt, bevorzugt sind jedoch PVC oder Vinylchloridenthaltende Copolymere.

Gegenstand der vorliegenden Erfindung ist auch eine Weichmacherzusammensetzung, die neben der C1- bis C5-Alkylester, vorzugsweise C4- bis C6-Alkylester, besonders bevorzugt C5-Alkylester der 1,2,3,4-Butantetracarbonsäure einen weiteren Weichmacher enthält. Je nach Anwendungszweck können ein oder mehrere zusätzliche, insbesondere von der erfindungsgemäßen Mischung von Tetraisopentylestern der 1,2,3,4-Butantetracarbonsäure verschiedene, Weichmacher in der Weichmacherzusammensetzung enthalten sein, um die Eigenschaften der resultierenden Weichmacherzusammensetzung gezielt einzustellen. Nach einer besonders bevorzugten Ausführungsform umfasst die Weichmacherzusammensetzung jedoch weniger als 5 Gew.-%, weiterhin bevorzugt weniger als 0,5 Gew.-%, besonders bevorzugt weniger als 0,1 Gew.-% Phthalate.

Der zusätzliche Weichmacher in der erfindungsgemäßen Weichmacherzusammensetzung kann aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, Cyclohexandicarboxylaten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Isophthalaten, Trimellitaten und oligomeren oder polymeren Estern auf Basis von Adipin-, Bernstein- oder Sebacinsäure, ausgewählt werden. In einer bevorzugten Ausführungsform der vorliegenden Erfindung enthält die Weichmacherzusammensetzung einen weiteren Weichmacher, der aus der Gruppe, bestehend aus Alkylbenzoaten, Alkylsulfonsäureestern des Phenols, Dialkyladipaten, Glycerinestern, C4-C6-Säuren von Polyolen, Citronensäuretrialkylestern, acetylierten Citronensäuretrialkylestern, Glykoldibenzoaten, Trialkylestern der Trimellitsäure, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate, und Dialkylestern der 1,2-, 1,3- oder 1,4-Cyclohexandicarbonsäure, ausgewählt wird.

In einer weiterhin bevorzugten Ausführungsform wird der weitere Weichmacher, der in der Weichmacherzusammensetzung enthalten ist, aus der Gruppe, bestehend aus C8- bis C13-Alkylbenzoaten, C4- bis C10-Dialkyladipaten, Pentaerythrit-tetravalerat, acetylierten Citronensäuretrialkylestern mit C4 bis C9-Alkylgruppen, C4- bis C10-Trialkyltrimellitaten, C4- bis C9-Dialkylterephthalaten, C4- bis C13-Dialkylphthalaten, insbesondere C9- bis C13-Dialkylphthalaten und C4- bis C10-Dialkylestern der 1,2-, 1,3- oder 1,4-Cyclohexandicarbonsäure, ausgewählt.

Gegenstand der vorliegenden Erfindung ist daher auch eine Kunststoffzusammensetzung, enthaltend die erfindungsgemäßen C1- bis C5-Alkylester, vorzugsweise C4- bis C6-Alkylester, besonders bevorzugt C5-Alkylester der der 1,2,3,4-Butantetracarbonsäure oder die Weichmacherzusammensetzung und ein oder mehrere Polymere.

Geeignete Polymere sind vorzugsweise ausgewählt aus der Gruppe, die gebildet wird durch PVC, Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikone.

In einer bevorzugten Ausführungsform ist mindestens ein Polymer bzw. sind vorzugsweise mindestens 90 Gew.-% der mehreren Polymere in der Weichmacherzusammensetzung ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und CoPolymeren von Vinylchlorid mit Vinylacetat oder mit Butylacrylat. Besonders bevorzugt ist hiervon PVC.

Die Menge an der erfindungsgemäßen Mischung von Tetraisopentylestern der 1,2,3,4-Butantetracarbonsäure oder der Weichmacherzusammensetzung in der Kunststoffzusammensetzung beträgt vorzugsweise 5 bis 150 Massenteile, bevorzugt 10 bis 120 Massenteile, besonders bevorzugt 15 bis 110 Massenteile und ganz besonders bevorzugt 20 bis 100 Massenteile pro 100 Massenteile Polymer. Es sind aber auch ein oder mehrere Polymere enthaltende Zusammensetzungen denkbar, die weniger als 20 Massenteile der C1- bis C5-Alkylester, vorzugsweise C4- bis C6-Alkylester, besonders bevorzugt C5-Alkylester der 1,2,3,4-Butantetracarbonsäure pro 100 Massenteile Polymer umfassen.

Ein weiterer bevorzugter Gegenstand der vorliegenden Erfindung ist eine Kunststoffzusammensetzung, die die C1- bis C5-Alkylester, vorzugsweise C4- bis C6-Alkylester, besonders bevorzugt C5-Alkylester der 1,2,3,4-Butantetracarbonsäure und einen schnell gelierenden Weichmacher ausgewählt aus der Gruppe, bestehend aus Dibutylterephthalat, Di(iso)-pentylterephthalat, Isodecylbenzoat, Isononylbenzoat, Acetyltributylcitrat, Tributylcitrat, Dipropylenglycoldibenzoat, Diethylenglycoldibenzoat, Triethylenglycoldibenzoat und Mischungen von zwei oder mehr davon, und mindestens ein Polymer, vorzugsweise PVC enthält.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Die vorliegende Erfindung wird nachfolgend anhand von Beispielen erläutert. Die Beispiele betreffen bevorzugte Ausführungsformen, sind aber nicht als die Erfindung einschränkend zu verstehen.

### Beispiele

### Herstellung der 1,2,3,4-Butantetracarbonsäure

Die Herstellung der 1,2,3,4-Butantetracarbonsäure wurde in einem Rührreaktor aus Glas mit Heizmantel und innenliegender Kühlschlange durchgeführt. Dazu wurden Tetrahydrophthalsäureanhydrid (456 g oder 3 mol) und Natriumwolframat Dihydrat (16,5 g oder 0,05 mol) in Wasser (1 L) im Reaktor vorgelegt, auf 65 °C aufgeheizt und für etwa 45 min. gerührt. Anschließend wird innerhalb von 40 Minuten eine 35%ige Wasserstoffperoxidlösung zugegeben (1457 g, 25% Überschuss) und die Reaktion beginnt. Die Temperatur wird während der Reaktion bei maximal 90 °C durch Gegenkühlung (Wasserkühlung durch die innere Kühlschlange) abgefangen und bis zum gewünschten Umsatz gehalten (etwa 6 Stunden).

Die Kristallisation der 1,2,3,4-Butantetracarbonsäure aus der Reaktionslösung erfolgt schon beim Abkühlen nach Beendigung. Die Reaktionslösung wurde dann in einem Rotationsverdampfer bei 60 °C durch Verdampfen des Wassers eingeengt. Die auskristallisierte 1,2,3,4-Butantetracarbonsäure wurde mittels Filtration abgetrennt und fiel als weißer Feststoff an.

### Herstellung von Tetraisopentylestern der 1,2,3,4-Butantetracarbonsäure

In eine Apparatur (Dreihalskolben, Rührer und Kühler mit Wasserauskreiser) wurden die wie vorgenannt hergestellte 1,2,3,4-Butantetracarbonsäure und eine 50:50 Mischung aus 2-Methylbutanol und n-Pentanol eingefüllt (25% Alkoholüberschuss) (2-Methylbutanol: Firma Sigma Aldrich, Reinheit ≥ 99 %; n-Pentanol: Firma Honeywell, Reinheit ≥ 99 %). Dazu wurde Tetra-n-Butyltitanat als Katalysator hinzugegeben (Molverhältnis von 1,2,3,4-Butantetracarbonsäure : Katalysator = 500 : 1) und die Reaktion gestartet. Die Reaktion verlief unter Einperlung von Stickstoff. Die Reaktanden wurden langsam bis zu einer Reaktionstemperatur von 200 °C erhitzt. Nach Erreichen der Reaktionstemperatur wurden zusätzlicher Alkohol zudosiert. Bei der Dosierung wurde darauf geachtet, dass die Reaktionstemperatur nicht unter 200°C fiel.

Im Verlauf der Veresterung entsteht kontinuierlich Wasser, welches mit dem Alkohol ein Azeotrop bildet. Das Azeotrop wird kondensiert und anschließend wird mit einem Wasserauskreiser das Wasser entfernt und der Alkohol wieder der Reaktion hinzugefügt. In regelmäßigen Abständen wurde der Reaktionsfortschritt über die SZ kontrolliert bis eine SZ von < 0,5 mg KOH pro g Probe erreicht wurde. Anschließend wird der überschüssige Alkohol bei 160 °C unter Vakuum abdestilliert. Nach weiterem Abkühlen auf 80 °C wird durch Zugabe von Lauge die restliche Säure neutralisiert und der Katalysator zerstört. Im letzten Schritt werden Feststoffe durch Filtration bei 80°C von dem Produkt abgetrennt.

## Patentansprüche

1. Verfahren zur Herstellung von 1,2,3,4-Butantetracarbonsäuretetraalkylestern, die Alkylgruppen mit 1 bis 6 Kohlenstoffatome aufweisen, wobei das Verfahren die folgenden Schritte umfasst:
a) Oxidieren von Tetrahydrophthalsäureanhydrid (THPA) in wässriger Lösung mit Wasserstoffperoxid unter Verwendung eines Katalysators, wodurch eine Reaktionslösung entsteht, die zumindest 1,2,3,4-Butantetracarbonsäure, nicht oder nur teilweise umgesetztes THPA und restliches Wasserstoffperoxid enthält,
b) Abtrennen der 1,2,3,4-Butantetracarbonsäure aus der Reaktionslösung, die in Schritt a) erhalten wurde;
c) Verestern der 1,2,3,4-Butantetracarbonsäure mit einem C1- bis C6-Alkohol in Abwesenheit oder Gegenwart eines Katalysators;
d) Abtrennen des bei der Veresterung entstehenden Wassers und des überschüssigen Alkohols unter Erhalt der Tetraalkylester der 1,2,3,4-Butantetracarbonsäure mit Alkylgruppen mit 1 bis 6 Kohlenstoffatome.

2. Verfahren nach Anspruch 2, wobei ein C4- bis C6-Alkohol bei der Veresterung eingesetzt wird, wodurch 1,2,3,4-Butantetracarbonsäuretetraalkylester entstehen, die Alkylgruppen mit 4 bis 6 Kohlenstoffatome aufweisen.

3. Verfahren nach Anspruch 1 oder 2, wobei als C4- bis C6-Alkohol 1-Butanol, 2-Butanol, 2-Methyl-1-propanol, 2-Methyl-2-propanol, 1-Pentanol, 2-Pentanol, 3-Pentanol, 2-Methyl-1-butanol, 2-Methyl-2-butanol, 3-Methyl-1-butanol, 3-Methyl-2-butanol, 2,2-Dimethyl-1-propanol, 1-Hexanol, 2-Hexanol, 3-Hexanol, 2-Methyl-1-pentanol, 3-Methyl-1-pentanol, 4-Methyl-1-pentanol, 2-Methyl-2-pentanol, 3-Methyl-2-pentanol, 4-Methyl-2-pentanol, 2-Methyl-3-pentanol, 3-Methyl-3-pentanol, 2,2-DiMethyl-1-butanol, 2,3-Di-Methyl-1-butanol, 3,3-Di-Methyl-1-butanol, 2,3-Di-Methyl-2-butanol, 3,3-Di-Methyl-2-butanol, 3-Ethyl-1-butanol oder Mischungen von zwei oder mehr davon eingesetzt werden, vorzugsweise 1-Pentanol, 2-Pentanol, 3-Pentanol, 2-Methyl-1-butanol, 2-Methyl-2-butanol, 3-Methyl-1-butanol, 3-Methyl-2-butanol, 2,2-Dimethyl-1-propanol oder Mischungen davon eingesetzt werden, besonders bevorzugt eine Mischung von mindestens zwei aus der Gruppe 1-Pentanol, 2-Methyl-1-butanol und 3-Methyl-1-butanol eingesetzt wird.

4. Verfahren nach einem der vorhergehenden Ansprüche, wobei Wasserstoffperoxid in einem Überschuss von 5 bis 50%, vorzugsweise 15 bis 40 %, besonders bevorzugt 20 bis 30 % zugegeben wird.

5. Verfahren nach einem der vorhergehenden Ansprüche, wobei als Katalysator bei der Oxidation in Schritt a) ein Katalysator, der Wolfram enthält, vorzugsweise Phosphorwolframsäure oder Natriumwolframat, besonders bevorzugt Natriumwolframat eingesetzt wird.

6. Verfahren nach einem der vorhergehenden Ansprüche, wobei die Abtrennung der 1,2,3,4-Butantetracarbonsäure in Schritt b) mittels Kristallation und anschließender Filtration erfolgt.

7. Verfahren nach Anspruch 6, wobei die 1,2,3,4-Butantetracarbonsäure als Filterkuchen zurückbleibt.

8. Verfahren nach Anspruch 6 oder 7, wobei die Kristallation der 1,2,3,4-Butantetracarbonsäure als Kühlungskristallisation oder als Verdampfungskristallisation durchgeführt wird.

9. Verfahren nach Anspruch 8, wobei die Kühlungskristallisation der 1,2,3,4-Butantetracarbonsäure bei einer Temperatur im Bereich von 2 bis 25 °C, vorzugsweise 3 bis 22 °C erfolgt.

10. Verfahren nach Anspruch 8, wobei die Verdampfungskristallisation der 1,2,3,4-Butantetracarbonsäure bei einer Temperatur im Bereich von 50 bis 70 °C, vorzugsweise 55 bis 65 °C und optional im Vakuum erfolgt.

11. Verfahren nach einem der Ansprüche 8 bis 10, wobei der der entgaste Reaktoraustrag einer Kühlungskristallisation oder Verdampfungskristallisation und anschließend einer Filtration unterworfen wird, wobei nach der Filtration ein auskristallisiertes Produkt und eine flüssige Mutterlauge entsteht, wovon die flüssige Mutterlauge zu dem oder zu den Reaktor(en) zurückgeführt wird.

12. Verfahren nach einem der Ansprüche 8 bis 11, wobei ein bei der Verdampfungskristallisation entstehender Wasserdampf zur Wärmeintegration insoweit verwendet, dass zunächst eine Kompression des Wasserdampfes auf ein höheres Druckniveau erfolgt und anschließend Energie vom komprimierten Wasserdampf in einem oder mehreren Wärmetauschern innerhalb des Prozesses, z. B. bei der Kristallisation zur Erwärmung der Kristallisationslösung, zur Erwärmung des Inertgases oder zur Erwärmung beim Trocknen, übertragen wird.

13. Verfahren nach einem der vorhergehenden Ansprüche, wobei bei der Veresterung in Schritt c) ein Inertgas zugeführt wird.

14. Verfahren nach einem der vorhergehenden Ansprüche, wobei die 1,2,3,4-Butantetracarbonsäure nach der Abtrennung in Schritt b) getrocknet wird bevor sie in der Veresterung in Schritt c) eingesetzt wird.

15. Verfahren nach einem der vorhergehenden Ansprüche, wobei der Veresterungskatalysator erst zur Veresterung in Schritt c) zugegeben wird, wenn der Wassergehalt der 1,2,3,4-Butantetracarbonsäure reduziert worden ist, vorzugsweise durch Aufheizen auf Temperaturen von größer oder gleich 100 °C.
